# EUROPEAN PATENT SPECIFICATION

(11) **EP 0 560 155 B1**
(45) Date of publication and mention of the grant of the patent: **08.05.1996**
(21) Application number: 93103207.2
(22) Date of filing: 01.03.1993
(51) Int. Cl.: C07D 209/48

(54) **Process for separating phthalimidoperoxycaproic acid (PAP) from solution(s) in organic solvents**
Verfahren für die Herstellung von Phthalimidoperoxycapronsäure (PAP) durch die Abtrennung von organischen Lösungsmitteln
Procédé pour la séparation de l'acide caproique de l'hydroxy-phthalimide (PAP) à partir d'une solution organique

(30) Priority: 10.03.1992 IT MI920539
(43) Date of publication of application: 15.09.1993
(73) Proprietor: AUSIMONT S.p.A., I-20121 Milano (IT)
(72) Inventor: Cavallotti, Claudio, I-20146 Milano (IT); Troglia, Claudio, I-20149 Milano (IT); Garaffa, Roberto, I-80127 Napoli (IT)
(74) Representative: Sama, Daniele, Dr.

(56) References cited:
- EP-A- 0 325 288
- EP-A- 0 490 409
- WO-A-90/07501
- WO-A-90/14336
- DE-A- 3 823 172
- DE-A- 4 003 309
- FR-A- 2 385 697

## Description

The present invention relates to a process for separating phthalimido-peroxycaproic acid (PAP) from solutions in organic solvents in which it is dissolved.

In particular, the present invention relates to a process for separating phthalimido-peroxycaproic acid (PAP) from its solutions in chlorohydrocarbons used in order to prepare it, and from the solutions in the other organic solvents used for its subsequent purification.

The process for preparing PAP is known from European Patent Application No. 490,409.

According to this process, phthalimido-caproic acid (PAC) is converted into the peroxyacid, by H₂O₂, in the presence of a strong acid, in a double-phase system, in the presence of an organic solvent constituted by a halogenated aliphatic chlorohydrocarbon selected from dichloromethane and trichloromethane. The resulting PAP is recovered from the organic phase by low-temperature crystallization, or solvent removal under vacuum.

PAP recovered in that way can be purified by recrystallization or stripping, using organic solvents as disclosed in Italian Patent Application No. MI92-A-000 381 (filed on Feb. 21st, 1992).

Patent application FR-A-2,385,697 describes an improved process for preparing a peroxycarboxylic acid by oxidation of the corresponding carboxylic acid with hydrogen peroxide in an aqueous phase in the presence of a strong acid as catalyst. The improvement consists in reducing reaction time by adding to the reaction mixture an inert solvent, such as methylene chloride, which is not soluble in water and is a good solvent for the peroxyacid, while the solvent capacity for the starting acid is scarce. The peroxyacid is recovered by separation of the organic phase, and subsequent crystallization of the peroxyacid from the solvent.

The processes which apply the usual separation methods display some disadvantages; for example, the organic solvent used are not completely recyclable, owing to the accumulation of impurities, or unavoidable losses in the case of vacuum evaporation.

The present Applicant found now that the organic solvents can be re-used by means of a novel process used as an alternative to the usual separation methods, such as low-temperature crystallization or vacuum evaporation. This novel process makes it possible the process to be carried out under maximum safety conditions.

Therefore, the subject-matter of the present invention is a process for separating phthalimido-peroxycaproic acid (PAP) from a solution of PAP in an organic solvent, said organic solvent having a solubility in water equal to or lower than 10% by weight, which comprises:
(a) forming a suspension by feeding the solution to a reactor containing an aqueous medium;
(b) removing the organic solvent by bubbling air or an inert gas into the resulting suspension, at a temperature of from 20° to 40°C and at a pressure of from 0 to 200 mmHg (from 0 to 2.66·10⁻² MPa), and condensing the evaporated solvent in a condenser system; and
(c) recovering PAP by filtering the suspension.

More particularly, the subject-matter of the present invention is a process for separating phthalimido-peroxycaproic acid (PAP) from solutions in organic solvents, characterized in that said process comprises the following steps:
-- the solution of PAP in an organic solvent having a solubility in water which is equal to, or lower than, 10% by weight (1), is fed to a reactor (R) equipped with a stirring system and containing an aqueous medium (3), through which a stream of air or inert gas (2) is bubbled, at a temperature comprised within the range of from +20°C to 40°C, and under a gauge pressure comprised within the range of from 0 to 0.266 bars (from 0 to 200 mm_{H g});
-- the organic solvent is evaporated off from the resulting suspension, kept with stirring;
-- the evaporated solvent is recovered in a condenser system (C); and
-- PAP is recovered by filtering the suspension.

Following the feeding of organic PAP solution to the aqueous medium, a suspension is obtained which derives from the precipitation of PAP in the aqueous phase.

The ratio of the aqueous medium to suspended PAP is comprised within the range of from 6 to 15 by weight, preferably of from 8 to 12 by weight.

The temperature of the aqueous medium to which the PAP solution is fed is preferably comprised within the range of from +30°C to 35°C.

The gauge pressure inside the reactor (R) is preferably comprised within the range of from 0.133 to 0.2 bars (from 100 to 150 mm_{H g}).

Exemplary organic solvents for PAP, having a solubility in water equal to, or lower than, 10% by weight, and from which the separation of PAP is carried out, are chlorohydrocarbons, such as, e.g., dichloromethane and trichloromethane, aliphatic esters, such as, e.g., methyl acetate and ethyl acetate.

The aqueous medium can be constituted by demineralized water, or aqueous solutions of inorganic salts, such as, e.g., Na₂SO₄, MgSO₄, (NH₄)₂SO₄. If an aqueous solution of inorganic salts is used, the solution can have a concentration comprised within a wide range, preferably of from 10 to 40%. The aqueous medium can contain small amounts (0.1-3%) of substances acting as acidity neutralizers, or metal ion sequestering agents, such as, e.g., sodium or potassium salts of citric, tartaric, phosphoric, phthalic acids, or NaOH, Na₂CO₃, and so forth.

The inert gas used in the process can be, e.g., N₂, CO₂, and their mixtures.

The solvents of PAP solutions are recovered according to the process of the present invention, in a system of condensers (C) kept at a temperature not lower than 0°C, in order to prevent water entrained by the organic solvents, from solidifying. The system of condensers (C) continuously subtracts a portion of the organic solvents from the recycled gas, in such a way as to allow them to be completely recovered at the end of the operation.

According to the process of the invention, PAP is recovered by filtration from an aqueous medium, as a crystalline powder, or granular solid, and is subsequently washed with demineralized water and dried in a desiccator (CaCl₂).

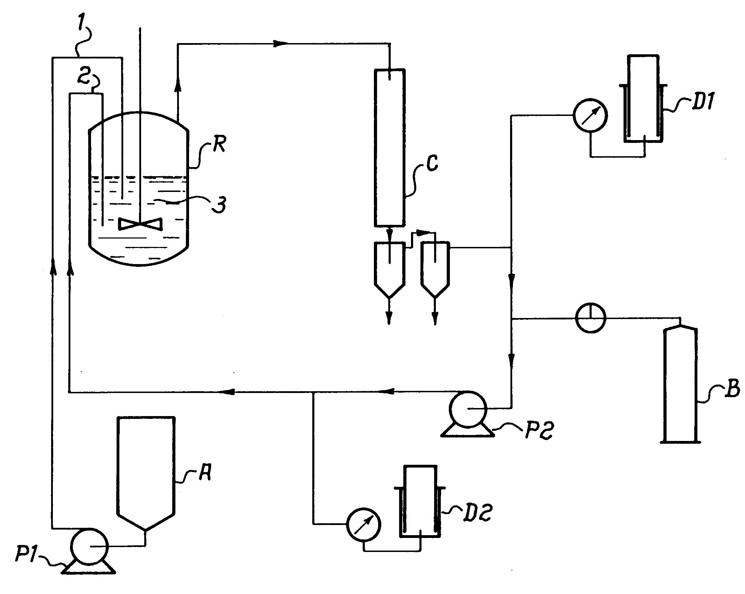

A preferred form of practical embodiment of the process of the invention is illustrated in the flow scheme shown in the accompanying drawing. According to the drawing, an organic solution of phthalimido-peroxycaproic acid (PAP) (1), coming from the feed tank (A), is fed continuously, by means of a metering pump (P₁), to the aqueous medium (3) kept stirred inside the reactor (R). In the aqueous medium a gas stream (2) is caused to bubble. The gas is partially constituted by inert gas drawn at the beginning of the test, from the tank (B), and partially by not condensed vapours, continuously recycled by the pump (P₂). The solvent is recovered by means of a system of condensers (C) kept at a temperature higher than 0°C (of round 5°C). The hydraulic valves equipped with pressure gauges (D₁, D₂) are used in order to keep constant the pressure inside the equipment, preventing vapour leakages to the surrounding atmosphere.

The process according to the present invention can also be performed continuously. The stirred PAP suspension can be drawn continuously, and the aqueous medium and PAP can be refilled.

The PAP separated by means of this process is of good purity. Its purity is comprised within the range of from 96 to 99% by weight.

The process makes it possible the organic solvents to be recovered with a rate of 98% and at such a purity level that they can be used again in the subsequent processes for PAP separation or purification. In such a way, the process according to the present invention is a novel separation method and can be adopted jointly with the process for PAP preparation as disclosed in European Patent Application No. 490,409.

Furthermore, the process of the present invention makes it possible the steps in question to be carried out under maximal safety conditions, both avoiding environmental pollution problems and all those problems which are connected with possible risks of fires or explosions owing to the possible flammability of the used organic solvents.

In order to better understand the present invention and to practice it, some illustrative examples thereof are reported in the following.

### Examples 1 - 3

A solution containing 400 g (18.8%) of PAP, 0.2% of H₂SO₄ and 80.5% of dichloromethane (deriving from the peroxidation process), was added to an aqueous medium, inside the reactor (R). The chlorinated solvent was removed through an air stream with vapours being recycled, and solvent vapours were condensed inside the system of condensers (C).

### Example 4

A solution containing 400 g (12.31%) of PAP and 87.5% of ethyl acetate, was added to an aqueous medium, inside the reactor (R). The organic solvent (i.e., the ester) was removed by stirring the obtained suspension, causing an N₂ stream to bubble through the suspension, and by recycling the not condensed solvent vapours.

### Examples 5 and 6 (Comparative Examples)

For comparison purposes, two tests were carried out in order to compare the separation methodology according to the present invention to the usual methodologies, such as, e.g., crystallization and vacuum evaporation.

A solution containing 400 g (18.8%) of PAP, and 80.5% of dichloromethane was submitted to crystallization at a temperature of -5°C in Example 5. The solution (296 g), obtained after filtration, contained 94.1% of dichloromethane and 5.9% of impurities due to the presence of phthalimido-peroxycaproic (PAP) and phthalimido-caproic acid (PAC).

According to Example 6, the solvent of the solution containing 400 g (14.98%) of PAP and 84.8% of ethyl acetate was evaporated under vacuum in a rotational evaporator at a temperatue of 37°C, under a residual pressure of 0.027 bars (20 mm_{H g} ).

## Claims

1. A process for separating phthalimido-peroxycaproic acid (PAP) from a solution of PAP in an organic solvent, said organic solvent having a solubility in water equal to or lower than 10% by weight, which comprises:
(a) forming a suspension by feeding the solution to a reactor containing an aqueous medium;
(b) removing the organic solvent by bubbling air or an inert gas into the resulting suspension, at a temperature of from 20° to 40°C and at a pressure of from 0 to 200 mmHg (from 0 to 2.66·10⁻² MPa), and condensing the evaporated solvent in a condenser system; and
(c) recovering PAP by filtering the suspension.

2. The process according to claim 1, wherein the organic solvent is selected from chlorohydrocarbons and aliphatic esters.

3. The process according to claim 2, wherein the organic solvent is selected from: dichloroethane, trichloroethane, methyl acetate, and ethyl acetate.

4. The process according to anyone of the previous claims, wherein the inert gas is selected from N₂, CO₂, and mixtures thereof.

5. The process according to anyone of the previous claims, wherein during step (b) the suspension is stirred.

6. The process according to anyone of the previous claims, wherein step (b) is carried out at a pressure of from 100 to 150 mmHg (from 1.33·10⁻² to 2.00·10⁻² MPa).

7. The process according to anyone of the previous claims, wherein the ratio of the aqueous medium to the suspended PAP is from 6 to 15% by weight.

8. The process according to anyone of the previous claims, wherein step (a) is carried out at a temperature of from 30° to 35°C.

9. The process according to anyone of the previous claims, wherein the aqueous medium is an aqueous solution of inorganic salts.

10. The process according to claim 9, wherein the inorganic salts are selected from: Na₂SO₄, MgSO₄, (NH₄)₂SO₄, and mixtures thereof.

## Patentansprüche

1. Verfahren zur Abtrennung von Phthalimidoperoxycapronsäure (PAP) aus einer Lösung von PAP in einem organischen Lösungsmittel, wobei das organische Lösungsmittel eine Löslichkeit in Wasser von gleich oder weniger als 10 Gewichts-% aufweist, umfassend:
(a) Bildung einer Suspension durch Einführung der Lösung in einen Reaktor, der ein wäßriges Medium enthält;
(b) Entfernung des organischen Lösungsmittels durch Einblasen von Luft oder einem Inertgas in die resultierende Suspension bei einer Temperatur von 20 bis 40°C und bei einem Druck von 0 bis 200 mmHg (von 0 bis 2,66 x 10⁻² MPa) und Kondensieren des verdampften Lösungsmittels in einem Kondensator-System; und
(c) Isolierung von PAP durch Filtrieren der Suspension.

2. Verfahren nach Anspruch 1, in welchem das organische Lösungsmittel aus Chlorkohlenwasserstoffen und aliphatischen Estern ausgewählt ist.

3. Verfahren nach Anspruch 2, in welchem das organische Lösungsmittel aus Dichlorethan, Trichlorethan, Methylacetat und Ethylacetat ausgewählt ist.

4. Verfahren nach irgendeinem der vorangehenden Ansprüche, in welchem das Inertgas aus N₂, CO₂ und Mischungen davon ausgewählt ist.

5. Verfahren nach irgendeinem der vorangehenden Ansprüche, in welchem während Stufe (b) die Suspension gerührt wird.

6. Verfahren nach irgendeinem der vorangehenden Ansprüche, in welchem Stufe (b) bei einem Druck von 100 bis 150 mmHg (1,33 x 10⁻² bis 2,00 x 10⁻² MPa) durchgeführt wird.

7. Verfahren nach irgendeinem der vorangehenden Ansprüche, in welchem das Verhältnis des wäßrigen Mediums zur suspendierten PAP 6 bis 15 Gewichts-% beträgt.

8. Verfahren nach irgendeinem der vorangehenden Ansprüche, in welchem Stufe (a) bei einer Temperatur von 30 bis 35°C durchgeführt wird.

9. Verfahren nach irgendeinem der vorangehenden Ansprüche, in welchem das wäßrige Medium eine wäßrige Lösung von anorganischen Salzen ist.

10. Verfahren nach Anspruch 9, in welchem die anorganischen Salze aus Na₂SO₄, MgSO₄, (NH₄)₂SO₄ und Mischungen davon ausgewählt sind.

## Revendications

1. Procédé de séparation d'acide phtalimido-peroxycaproïque (PAP) à partir d'une solution de PAP dans un solvant organique, ledit solvant organique ayant une solubilité dans l'eau égale ou inférieure à 10% en poids, lequel procédé comprend les opérations consistant à :
(a) former une suspension par introduction de la solution dans un réacteur contenant un milieu aqueux ;
(b) retirer le solvant organique par barbotage d'air ou d'un gaz inerte dans la suspension résultante, à une température de 20° à 40°C et sous une pression de 0 à 200 mmHg (de 0 à 2,66 x 10⁻² MPa), et condenser le solvant évaporé dans un système de condenseurs ; et
(c) récupérer le PAP par filtration de la suspension.

2. Procedé selon la revendication 1, dans lequel le solvant organique est choisi parmi les chlorohydrocarbures et les esters aliphatiques.

3. Procedé selon la revendication 2, dans lequel le solvant organique est choisi parmi : le dichloroéthane, le trichloroéthane, l'acétate de méthyle et l'acétate d'éthyle.

4. Procédé selon l'une quelconque des revendications précédentes, dans lequel le gaz inerte est choisi parmi N₂, CO₂ et leurs mélanges.

5. Procédé selon l'une quelconque des revendications précédentes, dans lequel, pendant l'étape (b), la suspension est agitée.

6. Procédé selon l'une quelconque des revendications précédentes, dans lequel l'étape (b) est conduite sous une pression de 100 à 150 mmHg (de 1,33 x 10⁻² à 2,00 x 10⁻² MPa).

7. Procédé selon l'une quelconque des revendications précédentes, dans lequel le rapport du milieu aqueux au PAP en suspension est de 6 à 15% en poids.

8. Procédé selon l'une quelconque des revendications précédentes, dans lequel l'étape (a) est conduite à une température de 30°C à 35°C.

9. Procédé selon l'une quelconque des revendications précédentes, dans lequel le milieu aqueux est une solution aqueuse de sels minéraux.

10. Procédé selon la revendication 9, dans lequel les sels minéraux sont choisis parmi : Na₂SO₄, MgSO₄, (NH₄)₂SO₄ et leurs mélanges.
